# EUROPEAN PATENT SPECIFICATION

(11) **EP 0 488 812 B1**
(45) Date of publication and mention of the grant of the patent: **09.06.1999**
(21) Application number: 91311157.1
(22) Date of filing: 29.11.1991
(51) Int. Cl.: C12N 15/40, C12Q 1/70, C07K 14/18, G01N 33/576

(54) **Non-A, non-B hepatitis related nucleic acids, antigens, antibodies and their detection reagents**
Non-A, non-B hepatitisverwandte Nukleinsäuren, Antigene und Antikörper und ihre Feststellungsreagenzien
Acides nucléiques, antigènes et anticorps associés avec l'hépatite non-A, non-B et leurs réactifs de détection

(30) Priority: 30.11.1990 JP 33580690
(43) Date of publication of application: 03.06.1992
(73) Proprietor: IMMUNO JAPAN INC., Tokyo 167 (JP)
(72) Inventor: Nakamura, Tetsuo, Tokyo 167 (JP); Mishiro, Shunji, Tokyo (JP)
(74) Representative: Dean, John Paul

(56) References cited:
- EP-A- 0 318 216
- EP-A- 0 388 232
- EP-A- 0 441 644
- THE LANCET vol. 336, no. 8728, 8 December 1990, LONDON, GB pages 1400 - 1403; S. MISHIRO ET AL.: 'Non-A, non-B hepatitis specific antibodies directed at host-derived epitope: implication for an autoimmune process'

## Description

### Background to the Invention

The present invention relates to novel non-A, non-B hepatitis nucleic acid, protein, antigens and antibodies, and non-A, non-B hepatitis diagnostic reagents using such materials.

The causative agent of non-A, non-B hepatitis (hereinafter NANB hepatitis) afflicts human beings with such diseases as acute hepatitis, fulminant hepatitis, chronic hepatitis, liver cirrhosis, and hepatocellular carcinoma. Tn transfusion, it presents itself as the major cause of post-transfusion hepatitis (which occurs in approximately 10% of blood recipients in Japan). The causative viruses of hepatitis A and hepatitis B have already been isolated and these diseases are held under close medical control; however, the causative agent of NANB hepatitis has been left in mystery although its presence was assumed over 10 odd years ago.

In 1988, a research group at Chiron Corporation announced that they succeeded in cloning the gene of the causative agent of NANB hepatitis, and introduced an immunoassay kit for detection of antibody specific to NANB hepatitis. That diagnostic test is now being evaluated at various hospitals, research institutions and blood centers for its capability. According to the Chiron group, the causative agent is a Flavivirus because of its genomic structure, and they have named it hepatitis C virus (HCV). The data reported to date have confirmed a good correlation between HCV antibody detected by their immunoassay kit and the course of the disease of NANB hepatitis. With respect to the HCV antibody, however, there remain problems yet to be solved and elucidated: its insufficient specificity and sensitivity has been revealed in several reports, cross reaction between HCV and a large part of auto-immune hepatitis and hepatitis B, and proof that what they call HCV is the real causative agent of NANB hepatitis.

Non-A, non-B hepatitis specific diagnostic assays based on detection systems using the present invention in this and preceding applications (Japanese Patent Application Nos. 028191/90 dated February 9, 1990 and 153887/90 of June 14, 1990) are capable of detecting patients not detected by the kit using Chiron's antigen (Ortho HCV Ab ELISA test: Ortho Diagnostic Systems, Tokyo, Japan).

### Summary of the Invention

An object of the present invention is to provide previously unidentified nucleic acid, protein and peptides required for diagnosis of NANB hepatitis. Nucleic acid and protein under this invention have the usefulness in the diagnosis of NANB hepatitis and have been provided only by this invention as totally new materials.

Nucleotide sequences and amino acid sequences of nucleic acid and protein under this invention are in no way homologous to those of Chiron's HCV genome (European Patent Application No. 88310922.5.

The present invention discloses GOR gab DNA and GOR gab Protein having the following nucleotide and amino acid sequences respectively; detection assay for antibodies related to NANB hepatitis using such protein or peptides (containing 10 or more amino acids residue of such protein) as antigen; monoclonal or polyclonal antibodies reactive against such protein or peptides and methods of making such antibodies by immunization of animals with such protein or peptides; methods of using such protein or peptides to prepare such antibodies; diagnostic test kits for analyzing samples for NANB antigen or for the presence of antibodies directed against NANB; methods of detecting in a sample; NANB antigen or antigens directed against NANB; and detection assay for NANB hepatitis related antigen using such monoclonal and polyclonal antibodies. These detection assays can be used as efficient tools for diagnosis of NANB hepatitis.

### Brief Description of the Drawing

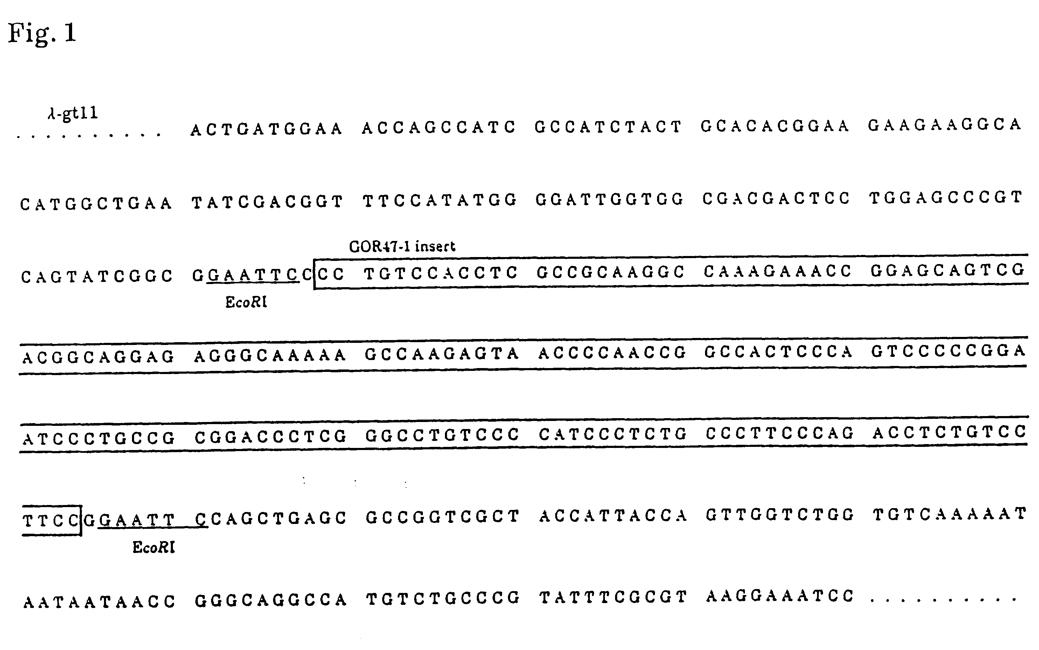

Figure 1 shows nucleotide sequence of recombinant lambda-gt11 phage DNA with GOR47-1 DNA inserted at the cleavage by E*coR*I (nucleotide sequence of GOR47-1 is shown in the box and that of lambda-gt11 phage DNA is shown on right and left sides of the box).

### Detailed Description of the Invention

GOR gab DNA has the following nucleotide sequence:

G A A T T C C C C    T G C C C T C T G A
T C A G C C T G C C    C A G A G C T T C G
G G C T C C G G G T    G C C C C A G A T G
C A C A A C C A G G    C C T C C G C A T T
T G T G G A C A T C    C A G G C G G A G C 100
C C C A G A A C A G    G G G T C C G G C G
G T G C C C C C A G    C G T G T C T C A A
G A T G G T G A C G    G A G G C G T C C T
A C T T C C C T G C    G C A G A G G G G A
T C G G C C T G C T    G C T T G C C A G C 200
C G C C C C A A G G    C T G A C A G A G A
G G C C C T C G G G    A G T C C G C A T C
T C A G C C C C C A    G G A A G A G G A A
G A C G A T C G C C    C A A T C T T C C A
G C C C T T G C T T    G G T C A C A G G T 300
T G C A C A G A T G    C C A A G A G A A C
C C G G G T G G C C    A G C A G C A G C C
A A C G C T C C A G    T G G C T C C A A G
G T C G G C A G A C    A G C C A G G G A A
G A C G C G C A A C    A G G T C A G G G A 400

T G G C A T G C A A G A C C A C C A C C
A C C A T C A G C T C T A A G C G A A T
C G T C C G T C G T C C A T C C C T A C
C G A G T T T G A A G A A A C C T A T T
A T C C T A A G A A G G T C T G G G T G 500
C C A A G T C C C C A C C G T C C T C C
G C C G A G G C T A T C T C C A A C T G
T T C A C C G A A G A G T G T C T C A A
G T T C T G C G C C T C C A A G C A G G
A G G C C G T G G A G A A G G C G C T G 600
A A C G A G G A G A A G G T G G C C T A
C G A C T G C A G C C C C A A C A A G A
A C A G G T A C C T G A A C G T G G T C
C T G A A C A C C C T C A A G A G A C T
G A A G G G C C T G A C C C C C A G C T 700
C C A T G C C G G G C C T C A G C A G G
G C C G C C C T G T A C A G C C G C C T
C C A G G A G T T C C T G C T C A G C C
A G G A C C A G C T C A A G G A G A A C
G G C T A C C C C T T C C C G C A C C C 800
C G A G C G G C C C G G A G G C G C C G
T C C T C T T C A C T G G C C A G G G G
A A G G G C C C C G G C G A C T C C T C
C T G C A G G G T C T G C T G C C G T T
G T G G C A C C G A G T A C C T G G T G 900
T C C T C C T C G G G C C G C T G T G T

A C G C G A C C A G    T T G T G T T A T T
A T C A C T G G G G    G C G G G T C C G C
T C G A G C C A G G    T G G C T G G A G G
C C G G G T T A G C    C A G T A C A C C T 1000
G C T G T G C A G C    T G C T C C T G G C
T C T G T G G G C T    G C C A G G T G G C
A A A G C A G C A C    G T G C G G G A C G
G C C G C A A G G A    C A G C C T C G A T
G G C T T C G T G G    A G A C C T T C A A 1100
G A A A G A G T T G    T C C A G A G A C G
C T T A T C C A G G    A A T C T A C G C C
T T G G A C T G T G    A G A T G T G C T A
C A C C A C G C A T    G G C C T A G A G C
T G A C C C G C G T    C A C C G T G G T G 1200
G A C G C C G A C A    T G C G A G T G G T
G T A C G A C A C C    T T C G T C A A G C
C C G A C A A C G A    G A T C G T G G A C
T A C A A C A C C A    G G T T T T C C G G
A G T C A C C G A G    G C C G A C G T C G 1300
C C A A G A C G A G    C A T C A C C T T G
C C C C A A G T G C    A A G C C A T C C T
G C T G A G C T T T    T T C A G C G C C C
A A A C C A T C C T    C A T C G G G C A C
A G C C T G G A G A    G C G A T C T G C T 1400
G G C C C T G A A G    C T C A T C C A C A
G C A C C G T G C T    G G A C A C G G C C

G T G C T C T T C C    C G C A C T A C C T
G G G T T T C C C C    T A C A A G C G T T
C C C T C A G G A A    T C T C G C G G C C 1500
G A C T A C C T G G    G A C A G A T C A T
C C A G G A C A G C    C A G G A C G G C C
A C A A G T G G A G    C G A G G A C G C A
A A C G C C T G C C    T G C A G C T G G T
G A T G T G G A A G    G T C C G A C A G C 1600
G C G C C C A G A T    C C A G C C A C G C
C A C C G G T C C G    C C T C T C C C G C
C G C C C T G G C C    T G T C C T T G G C
C C C A G G C C C C    T T C C A C A A C C
G C C A T C A G T C    C C G A G A G C T C 1700
A C C C T G T C C A    C C T C G C C G C A
A G G C C A A A G A    A A C C G G A G C A
G T C G A C G G C A    G G A G A G G G C A
A A A A G C C A A G    A G T A A C C C C A
A C C G G C C A C T    C C C A G T C C C C 1800
C G G A A T C C C T    G C C G C G G A C C
C T C G G G C C T G    T C C C C A T C C C
T C T G C C C T T C    C C A G A C C T C T
G T C C T T C C A C    T A A T C G C C T C
C C G C A G C A C C    G A G C C A C C A C 1900
T C C C A G T C C C    C C G A G T C C C T
G C C G C G C C C C    C T C G C G C C T G
T C C A C A T C C C    T C T G C C C A T C

C G A G A C C T C T G T C C T T A C A C
C A C T A G C C A C C C C A C G T G G G 2000
A C T T C C A T G G C C T C T G A G T A
C A A G G C C A G C C C C C C G G C C C
A C C A G C T T T C T G A A T G T G T G
C T T A C C T G T T 2070.

GOR gab Protein has the following amino acid sequence:

E F P L P S H Q P A     Q S F G L R V P Q M
H N Q A S A F V D I     Q A E P Q N R G P A
V P P A C L K M V T     E A S Y F P R Q R G
S A C C L P A A P R     L T E R P S G V R I
S A P R K R K T I A     Q S S S P C L V T G 100
C T D A K R T R V A     S S S Q R S S G S K
V G R Q P G K T R N     R S G M A C K T T T
T I S S K R I V R R     P S L P S L K K P I
I L R R S G C Q V P     T V L R R G Y L Q L
F T E E C L K F C A     S K Q E A V E K A L 200
N E E K V A Y D C S     P N K N R Y L N V V
L N T L K R L K G L     T P S S M P G L S R
A A L Y S R L Q E F     L L S Q D Q L K E N
G Y P F P H P E R P     G G A V L F T G Q G
K G P G D S S C R V     C C R C G T E Y L V 300
S S S G R C V R D Q     L C Y Y H W G R V R
S S Q V A G G R V S     Q Y T C C A A A P G

S V D C Q V A K Q H     V R D G R K D S L D
G F V E T F K K E L     S R D A Y P G I Y A
L D C E M C Y T T H     G L E L T R V T V V 400
D A D M R V V Y D T     F V K P D N E I V D
Y N T R F S G V T E     A D V A K T S I T L
P Q V Q A I L L S F     F S A Q T I L I G H
S L E S D L L A L K     L I H S T V L D T A
V L F P H Y L G F P     Y K R S L R N L A A 500
D Y L G Q I I Q D S     Q D G H N S S E D A
N A C L Q L V M W K     V R Q R A Q I Q P R
H R S A S P A A L A     C P W P Q A P S T T
A I S P E S S P C P     P R R K A K E T G A
V D G R R G Q K A K     S N P N R P L P V P 600
R N P C R G P S G L     S P S L C P S Q T S
V L P L I A S R S T     E P P L P V P R V P
A A P P R A C P H P     S A H P R P L S L H
H                 661.

In the nucleotide sequence of deoxyribonucleic acid, A, G, C and T stand for Adenine, Guanine, Cytosine and Thymine respectively.

In the amino acid sequence of protein, C, P, R, K, A, E, T, G, V, D, Q, S, N, L, I, M, F, Y, W and H stand for Cysteine, Proline, Arginine, Lysine, Alanine, Glutamic acid, Threonine, Glycine, Valine, Aspartic acid, Glutamine, Serine, Asparagine, Leucine, Isoleucine, Methionine, Phenylalanine, Tyrosine, Tryptophan and Histidine respectively.

Also disclosed is a detection assay for antibodies against NANB hepatitis using as antigen the cited protein, GOR gab Protein, or peptides containing 10 or more amino acids residue of such protein; monoclonal or polyclonal antibodies obtained by immunization of animals with such protein or peptides; and a detection assay for NANB hepatitis antigen using such monoclonal and polyclonal antibodies.

The following procedures were utilized:

### (1) Preparation of animal models for NANB hepatitis.

Various animals were injected with sera from donors which were known to have caused post transfusional NANB hepatitis in humans. Among these animals, only chimpanzees responded and expressed symptoms similar to human NANB hepatitis. In case of experimental NANB hepatitis in chimpanzees, some characteristic ultrastructural changes tend to appear in the cytoplasm of liver cells before clinical symptoms appear. Using this ultrastructural change as a reliable marker, experimental passage of the NANB causative agent was undertaken from a human being to a chimpanzee, then from that chimpanzee to other chimpanzees.

### (2) Materials for isolation of NANB hepatitis related gene.

Human serum (No. 30017) was injected into a chimpanzee (C37) and the serum from that chimpanzee in acute phase hepatitis was injected into five chimpanzees (C41, C43, C45, C46, C47). Plasma were taken form those five chimpanzees (when they were in the phase having the aforementioned ultrastructural changes) and pooled for injection to other animals including the chimpanzee CH19. Plasma taken from CH19 in acute phase of NANB hepatitis was used as the material for isolation of NANB hepatitis related gene.

### (3) Extraction of nucleic acids from chimpanzee plasma.

About six liters of plasma from the chimpanzee (CH19) in acute phase of NANB hepatitis was centrifuged on the J6 rotor (Beckman) at 3,000 rpm for 30 minutes and the supernatant was supercentrifuged on the Ti-15 rotor (Beckman) at 30,000 rpm for 5.3 hours at 4°C. The precipitate was suspended in 120 ml of 50 mM Tris-Cl (pH 7.5)/5mM EDT to obtain virus rich fraction concentrated by approximately 50 times. After additional to 6 ml of this fraction (equivalent to 300 ml of the original plasma) of 6 ml of SDS/Proteinase K cocktail (400 mM NaCl/20mM EDTA/4% SDS/100mM Tris-HCl buffer (pH 8.0)/Proteinase K 2 mg/ml) and overnight incubation at 37°C, nucleic acids were extracted with phenol and precipitated by ethanol.

### (4) cDNA synthesis.

Using ¼ in volume (equivalent to 75 ml of the original plasma) of the nucleic acids obtained under (3) above, cDNA was synthesized. After incubation of the template RNA at 65°C for three minutes, primary cDNA strand was synthesized in two tubes, one tube with oligo-dT₁₂ and the other with randam-hexanucleotide as primer. This series of cDNA synthesis, including synthesis of the secondary cDNA strand and blunting of double-stranded DNA termini, was conducted according to Cubler's method using a cDNA kit (Amersham, U.K.).

### (5) Preparation of cDNA libraries.

After protecting possible E*coR*I cleavage sites in the double-stranded cDNA prepared in (4) above with E*coR*I methylase, providing both ends with *Eco*RI linker, it was integrated into lambda-gt11 DNA at the E*coR*I site and assembled with phage protein to make recombinant phage. Series of this reaction was processed using the lambda-gt11 cloning kit (Amersham, U.K.). Library sizes of cDNA primed by oligo-dT₁₂ and by random-hexanucleotide were 1.0 x 10⁶ PFU and 4.3 x 10⁶ PFU respectively.

### (6) Screening of cDNA library.

cDNA libraries prepared under (5) were searched for a NANB hepatitis related cDNA clone by immune screening using two antibodies. After infecting E. coli Y1090 with the above recombinant phage, and dispensing it in LB-Agar plate for incubation at 43°C for three hours, a filter impregnated with IPTG was placed on it and incubated at 37°C for three hours. The filter was then removed and washed with the buffer and primary antibody was added to it. A mixture of plasma of a chimpanzee infected with NANB hepatitis, a human plasma known to have caused NANB hepatitis by needle stick accident, and a plasma of chronic NANB hepatitis patient were used as the primary antibody, and was incubated at 4°C overnight. After washing with the buffer and addition of the secondary antibody (peroxidase labeled anti-human IgG), it was incubated at room temperature for 30 minutes. It was then washed with the buffer and added with the mixture of DAB, Ni, Co, H₂O₂ for color development. If there were fractions of NANB related gene in the cDNA libraries, and if they were fused with the open reading frame (ORF) of β-galactosidase of lambda-gt11 phage in-frame, there should be fusion protein expressed by E. coli infected with the phage and, upon its recognition by NANB related antibody, it would bind to the antibody giving a positive signal. Inventors' experiment confirmed that point.

Among several clones giving a positive signals in the screening test, there was a clone named GOR47-1 described hereafter.

### (7) Purification of fusion protein made by NANB hepatitis related cDNA clone GOR47-1 and lambda-gt11 and β-galactosidase.

E. coli Y1089 was lysogenically infected with the NANB hepatitis related gene (GOR47-1) obtained under (6) above to make lysogen. (Lysogen was prepared according to the method described in "Constructing and Screening cDNA Libraries in lambda-gt11", Thanh V. Huyuh et al., DNA Cloning Vol. 1, a practical approach, ed. by D.M. Clover, 949-78, IRL Press, Oxford, 1985.) After culturing and inducing expression of protein by IPTG, and destruction of the lysogen by means of freezing-thawing and sonication, lysate was obtained and subjected to an affinity chromatography column (Sepharose 4B Immobilized IgG Fraction Rabbit anti-β-galactosidase: Cappel, U.S.A.) to obtain the fusion protein.

### (8) Detection of NANB hepatitis related antibody by the fusion protein of GOR47-1 and β-galactosidase.

The fusion protein obtained under (7) above was electrophoresed by SDS-PAGE and its pattern was transferred onto nitrocellulose membrane by the Western blotting technique. Sample plasma was applied onto this membrane and after incubation anti-human immune globulin labeled with peroxidase was added for detection of the antibody through color reaction.

The nitrocellulose membrane with the electrophoresed pattern transferred onto it was rinsed for ten minutes, naturally dried, shredded to strips 3.5mm wide, and those strips were immersed in TBS (50mM Tris-Cl buffer pH 7.4, 150mM NaCl) containing 2% skim milk at room temperature for one hour or at 4°C overnight (blocking), then washed with TBST (TBS containing 0.05% of Tween 20). After immersion at room temperature for one hour or at 4°C overnight in the primary antibody diluted 30 times with 3% BSA (primary antigen-antibody reaction), those strips were washed with TBST, immersed at room temperature for 30 minutes in biotinylated anti-human IgG or anti-human IgM (Vectastain ABC kit: Vector Laboratories, Inc., USA) diluted with TBS (secondary antigen-antibody reaction), washed with TBST, them immersed in peroxidase labeled mixture of biotin and avidin (Vectastain ABC kit: Vector Laboratories, Inc., USA) at room temperature for 30 minutes (biotin-avidin reaction), then washed with TBST followed finally by addition of coloring reagent to examine presence of reactive substances. The coloring reagent was prepared by adding 25 mg of DAB (3,3'-Diaminobenzidine tetrahydrochloride: Sigma, USA) to 50 ml of 50 µM NaPO₄ pH 7.4 and after dissolution of DAB, 1 ml of 5% CoCl₃ -6H₂O, 5%(NH₄)₂Ni(SO₄)₂ 6H₂O, then, 50 µl of 30% H₂O₂. When antibody was positive, a band of about 120K Dalton was stained dark blue.

Examples of the present invention are described below; however, they in no way limit the scope or extent of the present invention.

### Examples

### (1) GOR gab DNA.

The nucleotide sequence of NANB hepatitis related cDNA (GOR47-1) was determined in the following way.

GOR47-1 phage obtained in the above-mentioned (6) was purified, its DNA cleaved by E*coR*I to obtain cDNA, that cDNA subcloned to E*coR*I site of Phagescript (Stratagene, USA) and its nucleotide sequence determined by Sanger's method. Nucleotide sequence of the linking part of the insert arm of GOR47-1 phage DNA was similarly determined by the primer derived from the phage DNA. Those sequences revealed that GOR47-1 DNA was inserted in the lambda-gt11 DNA with the nucleotide sequence and direction shown in Figure 1, and insert DNA, that is GOR47-1 DNA, was cut out from DNA47-1 phage DNA. Figure 1 shows the nucleotide sequence of GOR47-1 together with its linking part with lambda-gt11 DNA. The sequence within the box is that of GOR47-1 and sequences on the right and left side of the box are those of lambda-gt11 DNA.

GOR47-1 DNA has the following nucleotide sequence:

C C T G T C C A C C     T C G C C G C A A G
G C C A A A G A A A     C C G G A G C A G T
C G A C G G C A G G     A G A G G C A A A
A A G C C A A G A G     T A A C C C C A A C
C G G C C A C T C C     C A G T C C C C G 100
G A A T C C C T G C     C G C G G A C C C T
C G G G C C T G T C     C C C A T C C C T C

T G C C C T T C C G     A G A C C T C T G T
C C T T C C     166

In the similar way, GOR47-1 DNAC (having DNA complementary to strain GOR47-1 DNA) was cut out from GOR47-1 phage DNA.

GOR47-1 DNAC has the following nucleotide sequence:

G G A A G G A C A G     A G G T C T G G G A
A G G G C A G A G G     G A T G G G A C A
G G C C C G A G G G     T C C G C G G C A G
G G A T T C C G G G     G G A C T G G G A G
T G G C C G G T T G     G G G T T A C T C T 100
T G G C T T T T T G     C C C T C T C C T G
C C G T C G A C T G     C T C C G G T T T C
T T T G G C C T T G     C G G C G A G G T G
G A C A G G     166

In the next step, GOR47-1 RNA and GOR47-1 RNAC were prepared from recombinant phage script of GOR47-1 DNAC using T₃ and T₇ promoters, they were labeled with radioisotope and used as probe to search the cDNA libraries prepared in (5) above, and GOR gab DNA, clone of cDNA, overlapping with but having longer sequence than GOR47-1, was obtained. The nucleotide sequence of GOR gab DNA was determined in the same way as above.

It is well known in the art that one or more nucleotides in a DNA sequence can be replaced by other nucleotides in order to produce the same protein. The present invention also concerns such nucleotide substitutions which yield a DNA sequence which codes for GOR gab Protein (the amino acid sequence of which is described above).

### (2) GOR gab Protein.

From GOR gab DNA, GOR gab Protein coded by it was obtained. Lambda-gt11 phage DNA used for preparation of cDNA libraries form which GOR gab DNA was derived is a so called "expression" vector and expresses fusion protein of β-galactosidase and cDNA derived protein by assembling cDNA in operon of IacZ gene of lambda-gt11. Among possible reading frames of GOR gab DNA, only one reading frame fuses in frame with open reading frame (ORF) of β-galactosidase of lambda-gt11.

Amino acids sequence of GOR gab Protein was shown above. It is well known in the art that one or more amino acids in an amino acid sequence can be replaced by equivalent other amino acids, as demonstrated by U.S. Patent No. 4,737,487 which is incorporated by reference, in order to produce an analog of the amino acid sequence. Any analogs of GOR gab Protein of the present invention involving amino acid deletions, amino acid replacements, such as replacements by other amino acids, or by isosteres (modified amino acids that bear close structural and spatial similarity to protein amino acids), amino acid additions, or isosteres additions can be utilized, so long as the sequences elicit antibodies recognizing NANB antigens.

In this invention, β-galactosidase can be substituted by such expression proteins as alkaline phosphatase and superoxide dismutase.

### (3) NANB hepatitis antigen related epitope by synthetic peptide based on the amino acid sequence coded for by GOR gab DNA.

Synthetic peptides of various lengths and for various regions based on the ORF of GOR gab DNA were prepared and examined for reactivity with NANB hepatitis related antibody to determined the location of NANB hepatitis epitope. Polystyrene microplates coated with synthetic peptide prepared by Merrifield's solid phase method as solid phase, sample sera as primary antibody, and peroxidase labeled anti-human IgG or IgM antibodies as secondary antibody for color reaction were used.

As a result, it has been found that peptides containing 10 or more amino acids residues have the antigen epitope. Any peptide sequence containing 10 or more consecutive amino acids can be utilized so long as the sequences elicit antibodies recognizing NANB antigens.

### (4) NANB hepatitis antibody detection system using protein and peptide described above as antigen.

50 µl each of GOR gab Protein or peptides having 10 or more amino acids residues dissolved in Tris-HCl buffer (10mM, pH 7.5) and adjusted to 5 µg/ml concentration was dispensed in each well on Costar vinyl plates (Toyobo, Japan) and incubated overnight at room temperature (coating of peptide). After washing the wells (0.1% Tween 20, 150mM NaCl; all washing procedures hereafter in this experiment, unless otherwise stated, were with this solution), 100 µl each of the blocking solution (0.1% Tween 20, 150mM NaCl, 30% fetal calf serum) was dispensed in the wells for overnight incubation at 4°C on a vibration free. After washing the wells, 50µl each of sample plasma, or serum preliminary diluted 30 times with the above-mentioned blocking solution, was dispensed in the wells for incubation at room temperature for 30 minutes on a vibrator. After washing the wells, 50µl each of peroxidase labeled anti-human IgG or IgM mouse monoclonal antibody solution was dispensed in the wells for incubation at room temperature for 30 minutes on a vibrator, then peroxidase substrate solution was added for color development and absorbance measurement at wavelength 492nm on a spectrophotometer.

When samples from 100 chronic liver disease patients (mixture of hepatitis B and NANB hepatitis) were assayed by EIA method, frequency by the assay using the protein or peptides under this invention turned out to be high for NANB chronic hepatitis, liver cirrhosis and hepatocellular carcinoma, while low for chronic liver disease, liver cirrhosis and hepatocellular carcinoma caused by hepatitis B virus, lupoid hepatitis of which autoimmune disorder was suspected, and primary biliary cirrhosis. The assay also showed low frequency for samples from normal subjects, thus proving the efficiency of the protein and peptides under the present invention for use in detection system of NANB hepatitis related antibody.

### (5) Preparation of monoclonal and polyclonal antibodies.

Specific monoclonal and polyclonal antibodies were obtained by immunizing such animals as mice, guinea pigs, rabbits, goats and horses with GOR gab Protein and the synthetic peptides bearing NANB hepatitis antigenic epitope described in the above example.

### (6) Antigen detection assay using antibody specific to NANB hepatitis related antigen.

By staining tissue sections of liver, etc. from NANB hepatitis patients with the aforementioned specific antibody labeled labeled with FITC as probe, the presence and locality of NANB hepatitis specific antigen in tissue was examined. By labeling the specific antibody with peroxidase or biotin, assay system for detection of NANB related antigen in patient serum or plasma was developed. The system was the sandwich method in which polystyrene microplates or beads coated with NANB hepatitis specific antibody were used as solid phase and after addition of samples for reaction with the solid phase, labeled specific antibody was added for the second reaction.

Experiments of those assays confirmed the effectiveness of monoclonal and polyclonal antibodies under the present invention for detection of NANB hepatitis related antigen.

The antigens and specific antibodies of the present invention provide diagnostic reagents capable of quicker and wider range of diagnosis of NANB hepatitis than any other conventional reagents, and can be used at blood centers, blood derivatives manufacturers, transfusion departments of hospitals and many others places for elimination of blood carrying the NANB hepatitis agent from transfusion blood and blood derivatives. Screening tests by the diagnostic reagents using the antigen and antibodies of the present invention will provide optimum means for prevention of post transfusion hepatitis which has long been a grave concern.

The nucleic acids and proteins of the present invention are manufactured not only as important reagents for serological and virological study of the NANB hepatitis causative agent and for pathological study of the disease caused by it, but also as indispensable materials for manufacturer of the aforementioned antigens and antibodies.

U.S. Patents US 5 077 193 and US 5 176 994 are hereby incorporated by reference for its teachings that the materials of the present invention may be utilized in detection kits and as vaccines.

## Claims

1. Recombinant GOR gab DNA consisting of the following nucleotide sequence:

GAATTCCCCC TGCCCTCTGA
TCAGCCTGCC CAGAGCTTCG
GGCTCCGGGT GCCCCAGATG
CACAACCAGG CCTCCGCATT
TGTGGACATC CAGGCGGAGC 100
CCCAGAACAG GGGTCCGGCG
GTGCCCCCAG CGTGTCTCAA
GATGGTGACG GAGGCGTCCT
ACTTCCCTGC GCAGAGGGGA
TCGGCCTGCT GCTTGCCAGC 200
CGCCCCAAGG CTGACAGAGA
GGCCCTCGGG AGTCCGCATC
TCAGCCCCCA GGAAGAGGAA
GACGATCGCC CAATCTTCCA
GCCCTTGCTT GGTCACAGGT 300
TGCACAGATG CCAAGAGAAC
CCGGGTGGCC AGCAGCAGCC
AACGCTCCAG TGGCTCCAAG
GTCGGCAGAC AGCCAGGGAA
GACGCGCAAC AGGTCAGGGA 400
TGGCATGCAA GACCACCACC
ACCATCAGCT CTAAGCGAAT

C G T C C G T C G T    C C A T C C C T A C
C G A G T T T G A A    G A A A C C T A T T
A T C C T A A G A [?]    G G T C T G G G T G 500
C C A A G T C C C C    A C C G T C C T C C
G C C G A G G C T A    T C T C C A A C T G
T T C A C C G A A G    A G T G T C T C A A
G T T C T G C G C C    T C C A A G C A G G
A G G C C G T G G A    G A A G G C G C T G 600
A A C G A G G A G A    A G G T G G C C T A
C G A C T G C A G C    C C C A A C A A G A
A C A G G T A C C T    G A A C G T G G T C
C T G A A C A C C C    T C A A G A G A C T
G A A G G G C C T G    A C C C C C A G C T 700
C C A T G C C G G G    C C T C A G C A G G
G C C G C C C T G T    A C A G C C G C C T
C C A G G A G T T C    C T G C T C A G C C
A G G A C C A G C T    C A A G G A G A A C
G G C T A C C C C T    T C C C G C A C C C 800
C G A G C G G C C C    G G A G G C G C C G
T C C T C T T C A C    T G G C C A G G G G
A A G G G G C C C G    G C G A C T C C T C
C T G C A G G G T C    T G C T G C C G T T
G T G G C A C C G A    G T A C C T G G T G 900
T C C T C C T C G G    G C C G C T G T G T
A C G C G A C C A G    T T G T G T T A T T
A T C A C T G G G G    G C G G G T C C G C

T C G A G C C A G G    T G G C T G G A G G
C C G G G T T A G C    C A G T A C A C C T 1000
G C T G T G [?] A G C    T G C T C C T G G C
T C T G T G G G C T    G C C A G G T G G C
A A A G C A G C A C    G T G C G G G A C G
G C C G C A A G G A    C A G C C T C G A T
G G C T T C G T G G    A G A C C T T C A A 1100
G A A A G A G T T G    T C C A G A G A C G
C T T A T C C A G G    A A T C T A C G C C
T T G G A C T G T G    A G A T G T G C T A
C A C C A C G C A T    G G C C T A G A G C
T G A C C C G C G T    C A C C G T G G T G 1200
G A C G C C G A C A    T G C G A G T G G T
G T A C G A C A C C    T T C G T C A A G C
C C G A C A A C G A    G A T C G T G G A C
T A C A A C A C C A    G G T T T T C C G G
A G T C A C C G A G    G C C G A C G T C G 1300
C C A A G A C G A G    C A T C A C C T T G
C C C C A A G T G C    A A G C C A T C C T
G C T G A G C T T T    T T C A G C G C C C
A A A C C A T C C T    C A T C G G G C A C
A G C C T G G A G A    G C G A T C T G C T 1400
G G C C C T G A A G    C T C A T C C A C A
G C A C C G T G C T    G G A C A C G G C C
G T G C T C T T C C    C G C A C T A C C T
G G G T T T C C C C    T A C A A G C G T T

C C C T C A G G A A    T C T C G C G G C C 1500
G A C T A C C T G G    G A C A G A T C A T
C C A G G A C A G C    C A G G A C G G C C
A C A A G T G G A G    C G A G G A C G C A
A A C G C C T G C C    T G C A G C T G G T
G A T G T G G A A G    G T C C G A C A G C 1600
G C G C C C A G A T    C C A G C C A C G C
C A C C G G T C C G    C C T C T C C C G C
C G C C C T G G C C    T G T C C T T G G C
C C C A G G C C C C    T T C C A C A A C C
G C C A T C A G T C    C C G A G A G C T C 1700
A C C C T G T C C A    C C T C G C C G C A
A G G C C A A A G A    A A C C G G A G C A
G T C G A C G G C A    G G A G A G G G C A
A A A A G C C A A G    A G T A A C C C C A
A C C G G C C A C T    C C C A G T C C C C 1800
C G G A A T C C C T    G C C G C G G A C C
C T C G G G C C T G    T C C C C A T C C C
T C T G C C C T T C    C C A G A C C T C T
G T C C T T C C A C    T A A T C G C C T C
C C G C A G C A C C    G A G C C A C C A C 1900
T C C C A G T C C C    C C G A G T C C C T
G C C G C G C C C C    C T C G C G C C T G
T C C A C A T C C C    T C T G C C C A T C
C G A G A C C T C T    G T C C T T A C A C
C A C T A G C C A C    C C C A C G T G G G 2000

ACTTCCATGG CCTCTGAGTA
CAAGGCCAGC CCCCCGGCCC
ACCAGCTTTC TGAATGTGTG
CTTACCTGTT 2070.

2. Recombinant GOR gab protein consisting of the following amino acid sequence:

EFPLPSHQPA | QSFGLRVPQM
HNQASAFVDI | QAEPQNRGPA
VPPACLKMVT | EASYFPRQRG
SACCLPAAPR | LTERPSGVRI
SAPRKRKTIA | QSSSPCLVTG | 100
CTDAKRTRVA | SSS QRSSGSK
VGTQPGKTRN | RSGMACKTTT
TISSKRIVRR | PSLPSLKKPI
ILRRSGCQVP | TVLRRGYLQL
FTEECLKFCA | SKQEAVEKAL | 200
NEEKVAYDCS | PNKNRYLNVV
LNTLKRLKGL | TPSSMPGLSR
AALYSRLQEF | LLSQDQLKEN
GUPFPHPERP | GGAVLFTGQG
KGPGDSSCRV | CCRCGTEYLV | 300
SSSGRCVRDQ | LCYYHWGRVR
SSQVAGGRVS | QYTCCAAAPG
SVDCQVAKQH | VRDGRKDSLD
GRVETFKKEL | SRDAYPGIYA

L D C E M C Y T T H     G L E L T R V T V V 400

D A D M R V V Y D T     F V K P D N E I V D

Y N T R F S G V T E     A D V A K T S I T L

P Q V Q A I L L S F     F S A Q T I L I G H

S L E S D L L A L K     L I H S T V L D T A

V L F P H Y L G F P     Y K R S L R N L A A 500

D Y L G Q I I Q D S     Q D G H N S S E D A

N A C L Q L V M W K     V R Q R A Q I Q P R

H R S A S P A A L A     C P W P Q A P S T T

A I S P E S S P C P     P R R K A K E T G A
      5o4
V D G R R G Q K A K     S N P N R P L P V P 600

R N P C R G P S G L     S P S L C P S Q T S

V L P L I A S R S T     E P P L P V P R V P

A A P P R A C P H P     S A H P R P L S L H

H               661.

3. A non-A, non-B hepatitis peptide comprising an amino acid chain of ten or more consecutive amino acids selected from amino acids numbered 1 to 564 of the protein according to claim 2.

4. A non-A, non-B hepatitis specific monoclonal or polyclonal antibody reactive with an antigen selected from antigens comprising amino acids numbered 1 to 564 of the protein sequence according to claim 2.

5. A non-A, non-B hepatitis specific monoclonal or polyclonal antibody reactive with an antigen comprising the peptide according to claim 3.

6. A non-A, non-B hepatitis diagnostic test kit for analyzing samples for the presence of antibodies directed against a non-A, non-B hepatitis antigen, comprising the non-A, non-B hepatitis antigen protein according to claim 2 attached to a solid substrate.

7. The non-A, non-B hepatitis diagnostic test kit according to claim 6, further comprising labeled anti-human IgG or IgM antibodies.

8. A non-A, non-B hepatitis diagnostic test kit for analyzing samples for the presence of antibodies directed against a non-A, non-B hepatitis antigen, comprising labeled anti-human IgG or IgM antibodies and the non-A, non-B hepatitis antigen peptide according to claim 3 attached to a solid substrate.

9. The non-A, non-B hepatitis diagnostic test kit according to claim 8, further comprising labeled anti-human IgG or IgM antibodies.

10. A non-A, non-B hepatitis diagnostic test kit for analyzing samples for the presence of a non-A, non-B hepatitis antigen, comprising the non-A, non-B hepatitis specific monoclonal or polyclonal antibody according to claim 4 attached to a solid substrate and said antibodies which are labelled.

11. A non-A, non-B hepatitis diagnostic test kit for analyzing samples for the presence of a non-A, non-B hepatitis antigen, comprising the non-A, non-B hepatitis specific monoclonal or polyclonal antibody according to claim 5 attached to a solid substrate and said antibodies which are labelled.

12. A method for detecting non-A, non-B hepatitis antibodies in a sample comprising:
(a) reacting said sample with the non-A, non-B hepatitis protein according to claim 2 attached to a solid substrate under conditions which allow the formation of an antigen-antibody complex;
(b) reacting said product of step (a) with labelled anti-human IgG or IgM antibodies; and
(c) detecting the presence of labelled anti-human IgG or IgM antibodies bound to said antigen-antibody complex.

13. A method for detecting non-A, non-B hepatitis antibodies in a sample comprising:
(a) reacting said sample with the non-A, non-B hepatitis peptide according to claim 3 attached to a solid substrate under conditions which allow the formation of an antigen-antibody complex;
(b) reacting said product of step (a) with labelled anti-human IgG or IgM antibodies; and
(c) detecting the presence of labelled anti-human IgG or IgM antibodies bound to said antigen-antibody complex.

14. A method for detecting non-A, non-B hepatitis antigens in a sample comprising:
(a) reacting said sample with the non-A, non-B hepatitis specific monoclonal or polyclonal antibody according to claim 4 attached to a solid substrate under conditions which allow the formation of an antigen-antibody complex;
(b) reacting the product of step (a) with the non-A, non-B hepatitis specific monoclonal or polyclonal antibody according to claim 4 which is labelled; and
(c) detecting the presence of labelled antibody.

15. A method for detecting non-A, non-B hepatitis antigens in a sample comprising:
(a) reacting said sample with the non-A, non-B hepatitis specific monoclonal or polyclonal antibody according to claim 5 attached to a solid substrate under conditions which allow the formation of an antigen-antibody complex;
b) reacting the product of step (a) with the non-A, non-B hepatitis specific monoclonal or polyclonal antibody according to claim 5 which is labelled; and
c) detecting the presence of labelled antibody.

16. The use of a protein according to claim 2 for the preparation of a non-A, non-B hepatitis specific polyclonal antibody reactive with a protein according to claim 2.

17. The use of a protein according to claim 2, in the production of a non-A, non-B hepatitis specific polyclonal antibody reactive with said protein.

18. The use of a protein according to claim 3 for the preparation of a non-A, non-B hepatitis specific polyclonal antibody reactive with a protein according to claim 3.

19. The use of a protein according to claim 3, in the production of a non-A, non-B hepatitis specific polyclonal antibody reactive with said protein.

## Patentansprüche

1. Rekombinante GOR-gab-DNS, bestehend aus der folgenden Nukleotidsequenz:

GAATTCCCCC TGCCCTCTGA
TCAGCCTGCC CAGAGCTTCG
GGCTCCGGGT GCCCCAGATG
CACAACCAGG CCTCCGCATT
TGTGGACATC CAGGCGGAGC 100
CCCAGAACAG GGGTCCGGCG
GTGCCCCCAG CGTGTCTCAA
GATGGTGACG GAGGCGTCCT
ACTTCCCTGC GCAGAGGGGA
TCGGCCTGCT GCTTGCCAGC 200
CGCCCCAAGG CTGACAGAGA
GGCCCTCGGG AGTCCGCATC
TCAGCCCCCA GGAAGAGGAA
GACGATCGCC CAATCTTCCA
GCCCTTGCTT GGTCACAGGT 300
TGCACAGATG CCAAGAGAAC
CCGGGTGGCC AGCAGCAGCC
AACGCTCCAG TGGCTCCAAG
GTCGGCAGAC AGCCAGGGAA
GACGCGCAAC AGGTCAGGGA 400
TGGCATGCAA GACCACCACC
ACCATCAGCT CTAAGCGAAT

C G T C C G T C G T    C C A T C C C T A C

C G A G T T T G A A    G A A A C C T A T T

A T C C T A A G A [?]    G G T C T G G G T G 500

C C A A G T C C C C    A C C G T C C T C C

G C C G A G G C T A    T C T C C A A C T G

T T C A C C G A A G    A G T G T C T C A A

G T T C T G C G C C    T C C A A G C A G G

A G G C C G T G G A    G A A G G C G C T G 600

A A C G A G G A G A    A G G T G G C C T A

C G A C T G C A G C    C C C A A C A A G A

A C A G G T A C C T    G A A C G T G G T C

C T G A A C A C C C    T C A A G A G A C T

G A A G G G C C T G    A C C C C C A G C T 700

C C A T G C C G G G    C C T C A G C A G G

G C C G C C C T G T    A C A G C C G C C T

C C A G G A G T T C    C T G C T C A G C C

A G G A C C A G C T    C A A G G A G A A C

G G C T A C C C C T    T C C C G C A C C C 800

C G A G C G G C C C    G G A G G C G C C G

T C C T C T T C A C    T G G C C A G G G G

A A G G G G C C C G    G C G A C T C C T C

C T G C A G G G T C    T G C T G C C G T T

G T G G C A C C G A    G T A C C T G G T G 900

T C C T C C T C G G    G C C G C T G T G T

A C G C G A C C A G    T T G T G T T A T T

A T C A C T G G G G    G C G G G T C C G C

T C G A G C C A G G     T G G C T G G A G G
C C G G G T T A G C     C A G T A C A C C T 1000
G C T G T G [?] A G C     T G C T C C T G G C
T C T G T G G G C T     G C C A G G T G G C
A A A G C A G C A C     G T G C G G G A C G
G C C G C A A G G A     C A G C C T C G A T
G G C T T C G T G G     A G A C C T T C A A 1100
G A A A G A G T T G     T C C A G A G A C G
C T T A T C C A G G     A A T C T A C G C C
T T G G A C T G T G     A G A T G T G C T A
C A C C A C G C A T     G G C C T A G A G C
T G A C C C G C G T     C A C C G T G G T G 1200
G A C G C C G A C A     T G C G A G T G G T
G T A C G A C A C C     T T C G T C A A G C
C C G A C A A C G A     G A T C G T G G A C
T A C A A C A C C A     G G T T T T C C G G
A G T C A C C G A G     G C C G A C G T C G 1300
C C A A G A C G A G     C A T C A C C T T G
C C C C A A G T G C     A A G C C A T C C T
G C T G A G C T T T     T T C A G C G C C C
A A A C C A T C C T     C A T C G G G C A C
A G C C T G G A G A     G C G A T C T G C T 1400
G G C C C T G A A G     C T C A T C C A C A
G C A C C G T G C T     G G A C A C G G C C
G T G C T C T T C C     C G C A C T A C C T
G G G T T T C C C C     T A C A A G C G T T

C C C T C A G G A A    T C T C G C G G C C 1500
G A C T A C C T G G    G A C A G A T C A T
C C A G G A C A G C    C A G G A C G G C C
A C A A G T G G A G    C G A G G A C G C A
A A C G C C T G C C    T G C A G C T G G T
G A T G T G G A A G    G T C C G A C A G C 1600
G C G C C C A G A T    C C A G C C A C G C
C A C C G G T C C G    C C T C T C C C G C
C G C C C T G G C C    T G T C C T T G G C
C C C A G G C C C C    T T C C A C A A C C
G C C A T C A G T C    C C G A G A G C T C 1700
A C C C T G T C C A    C C T C G C C G C A
A G G C C A A A G A    A A C C G G A G C A
G T C G A C G G C A    G G A G A G G G C A
A A A A G C C A A G    A G T A A C C C C A
A C C G G C C A C T    C C C A G T C C C C 1800
C G G A A T C C C T    G C C G C G G A C C
C T C G G G C C T G    T C C C C A T C C C
T C T G C C C T T C    C C A G A C C T C T
G T C C T T C C A C    T A A T C G C C T C
C C G C A G C A C C    G A G C C A C C A C 1900
T C C C A G T C C C    C C G A G T C C C T
G C C G C G C C C C    C T C G C G C C T G
T C C A C A T C C C    T C T G C C C A T C
C G A G A C C T C T    G T C C T T A C A C
C A C T A G C C A C    C C C A C G T G G G 2000

ACTTCCATGG CCTCTGAGTA
CAAGGCCAGC CCCCCGGCCC
ACCAGCTTTC TGAATGTGTG
CTTACCTGTT 2070.

2. Rekombinantes GOR-gab-Protein, bestehend aus der folgenden Aminosäuresequenz:

EFPLPSHQPA QSFGLRVPQM
HNQASAFVDI QAEPQNRGPA
VPPACLKMVT EASYFPRQRG
SACCLPAAPR LTERPSGVRI
SAPRKRKTIA QSSSPCLVTG 100
CTDAKRTRVA SSS QRSSGSK
VGTQPGKTRN RSGMACKTTT
TISSKRIVRR PSLPSLKKPI
ILRRSGCQVP TVLRRGYLQL
FTEECLKFCA SKQEAVEKAL 200
NEEKVAYDCS PNKNRYLNVV
LNTLKRLKGL TPSSMPGLSR
AALYSRLQEF LLSQDQLKEN
GUPFPHPERP GGAVLFTGQG
KGPGDSSCRV CCRCGTEYLV 300
SSSGRCVRDQ LCYYHWGRVR
SSQVAGGRVS QYTCCAAAPG
SVDCQVAKQH VRDGRKDSLD
GRVETFKKEL SRDAYPGIYA

L D C E M C Y T T H     G L E L T R V T V V 400

D A D M R V V Y D T     F V K P D N E I V D

Y N T R F S G V T E     A D V A K T S I T L

P Q V Q A I L L S F     F S A Q T I L I G H

S L E S D L L A L K     L I H S T V L D T A

V L F P H Y L G F P     Y K R S L R N L A A 500

D Y L G Q I I Q D S     Q D G H N S S E D A

N A C L Q L V M W K     V R Q R A Q I Q P R

H R S A S P A A L A     C P W P Q A P S T T

A I S P E S S P C P     P R R K A K E T G A
      564
V D G R R G Q K A K     S N P N R P L P V P 600

R N P C R G P S G L     S P S L C P S Q T S

V L P L I A S R S T     E P P L P V P R V P

A A P P R A C P H P     S A H P R P L S L H

H               661.

3. Ein Hepatitis(nicht -A oder -B)peptid, das eine Aminosäurekette aus 10 oder mehr aufeinanderfolgenden Aminosäuren, die aus den von 1 bis 564 durchnumerierten Aminosäuren des Proteins nach Anspruch 2 gewählt sind, umfaßt.

4. Ein hepatitis(nicht -A oder -B)spezifisches, monoklonales oder polyklonales Antikörperreagens mit einem Antigen, das aus den Antigenen gewählt ist, die die von 1 bis 564 durchnumerierten Aminosäuren der Proteinsequenz nach Anspruch 2 umfassen.

5. Ein hepatitis(nicht -A oder -B)spezifisches, monoklonales oder polyklonales Antikörperreagens mit einem Antigen, das das Peptid gemäß Anspruch 3 umfaßt.

6. Ein Hepatitis(nicht -A oder -B)diagnoseset zur Untersuchung von Proben auf die Anwesenheit von Antikörpern gegen ein Hepatitis(nicht -A oder -B)-Antigen, das das Hepatitis(nicht -A oder -B)-Antigenprotein gemäß Anspruch 2 auf einem festen Substrat umfaßt.

7. Ein Hepatitis(nicht -A oder -B)diagnoseset gemäß Anspruch 6, das weiterhin markierte anti-menschliche IgG- oder IgM-Antikörper umfaßt.

8. Ein Hepatitis(nicht -A oder -B)diagnoseset zur Untersuchung von Proben auf die Anwesenheit von Antikörpern gegen ein Hepatitis(nicht -A oder -B)-Antigen, das markierte anti-menschliche IgG- oder IgM-Antikörper und das Hepatitis(nicht -A oder -B)antigenpeptid gemäß Anspruch 3 auf einem festen Substrat umfaßt.

9. Ein Hepatitis(nicht -A oder -B)diagnoseset gemäß Anspruch 8, das weiterhin markierte anti-menschliche IgG- oder IgM-Antikörper umfaßt.

10. Ein Hepatitis(nicht -A oder -B)diagnoseset zur Untersuchung von Proben auf die Anwesenheit von einem Hepatitis(nicht -A oder -B)antigen, das einen hepatitis(nicht -A oder -B)spezifischen, monoklonalen oder polyklonalen Antikörper gemäß Anspruch 4 auf einem festen Substrat und die besagten markierten Antikörper umfaßt.

11. Ein Hepatitis(nicht -A oder -B)diagnoseset zur Untersuchung von Proben auf die Anwesenheit von einem Hepatitis(nicht -A oder -B)antigen, das einen hepatitis(nicht -A oder -B)spezifischen, monoklonalen oder polyklonalen Antikörper gemäß Anspruch 5 auf einem festen Substrat und die besagten markierten Antikörper umfaßt.

12. Verfahren zum Nachweis von Hepatitis(nicht -A oder -B)antikörpern in einer Probe, das folgendes umfaßt:
(a) die Umsetzung der besagten Probe mit dem Hepatitis(nicht -A oder -B)protein gemäß Anspruch 2 auf einem festen Substrat, unter Bedingungen, die die Bildung eines Antigen-Antikörperkomplexes zulassen;
(b) die Umsetzung des besagten Produkts aus Schritt (a) mit markierten anti-menschlichen IgG- oder Igm-Antikörpern; und
(c) den Nachweis der Anwesenheit von markierten anti-menschlichen IgG- oder IgM-Antikörpern, die an den besagten Antigen-Antikörperkomplex gebunden sind.

13. Verfahren zum Nachweis von Hepatitis(nicht -A oder -B)antikörpern in einer Probe, das folgendes umfaßt:
(a) die Umsetzung der besagten Probe mit dem Hepatitis(nicht -A oder -B)protein gemäß Anspruch 3 auf einem festen Substrat, unter Bedingungen, die die Bildung eines Antigen-Antikörperkomplexes zulassen;
(b) die Umsetzung des besagten Produkts aus Schritt (a) mit markierten anti-menschlichen IgG- oder IgM-Antikörpern; und
(c) den Nachweis der Anwesenheit von markierten anti-menschlichen IgG- oder IgM-Antikörpern, die an den besagten Antigen-Antikörperkomplex gebunden sind.

14. Verfahren zum Nachweis von Hepatitis(nicht -A oder -B)antigenen in einer Probe, das folgendes umfaßt:
(a) die Umsetzung der besagten Probe mit einem hepatitis(nicht -A oder -B)spezifischen, monoklonalen oder polyklonalen Antikörper gemäß Anspruch 4 auf einem festen Substrat, unter Bedingungen, die die Bildung eines Antigen-Antikörperkomplexes zulassen;
(b) die Umsetzung des Produkts aus Schritt (a) mit dem markierten hepatitis(nicht -A oder -B)spezifischen monoklonalen oder polyklonalen
Antikörper gemäß Anspruch 4; und (c) den Nachweis der Anwesenheit markierten Antikörpers.

15. Verfahren zum Nachweis von Hepatitis(nicht -A oder -B)-Antigenen in einer Probe, das folgendes umfaßt:
(a) die Umsetzung der besagten Probe mit dem hepatitis(nicht -A oder -B)spezifischen, monoklonalen oder polyklonalen Antikörper gemäß Anspruch 5 auf einem festen Substrat, unter Bedingungen, die die Bildung eines Antigen-Antikörperkomplexes zulassen;
(b) die Umsetzung des Produkts aus Schritt (a) mit dem markierten hepatitis(nicht -A oder -B)spezifischen monoklonalen oder polyklonalen Antikörper gemäß Anspruch 5; und
(c) den Nachweis der Anwesenheit markierten Antikörpers.

16. Die Verwendung eines Proteins gemäß Anspruch 2 zur Zubereitung eines hepatitis(nicht -A oder -B)spezifischen polyklonalen Antikörpers, der mit einem Protein gemäß Anspruch 2 reagiert.

17. Die Verwendung eines Proteins gemäß Anspruch 2 zur Herstellung eines hepatitis(nicht -A oder -B)spezifischen polyklonalen Antikörpers, der mit besagtem Protein reagiert.

18. Die Verwendung eines Proteins gemäß Anspruch 3 zur Zubereitung eines hepatitis(nicht -A oder -B)spezifischen polyklonalen Antikörpers, der mit einem Protein gemäß Anspruch 3 reagiert.

19. Die Verwendung eines Proteins gemäß Anspruch 3 zur Herstellung eines hepatitis(nicht -A oder -B)spezifischen polyklonalen Antikörpers, der mit besagtem Protein reagiert.

## Revendications

1. ADN GOR gab recombinant consistant en la séquence de nucléotides suivante :

G A A T T C C C C C    T G C C C T C T G A
T C A G C C T G C C    C A G A G C T T C G
G G C T C C G G G T    G C C C C A G A T G
C A C A A C C A G G    C C T C C G C A T T
T G T G G A C A T C    C A G G C G G A G C 100
C C C A G A A C A G    G G G T C C G G C G
G T G C C C C C A G    C G T G T C T C A A
G A T G G T G A C G    G A G G C G T C C T
A C T T C C C T G C    G C A G A G G G G A
T C G G C C T G C T    G C T T G C C A G C 200
C G C C C C A A G G    C T G A C A G A G A
G G C C C T C G G G    A G T C C G C A T C
T C A G C C C C C A    G G A A G A G G A A
G A C G A T C G C C    C A A T C T T C C A
G C C C T T G C T T    G G T C A C A G G T 300
T G C A C A G A T G    C C A A G A G A A C
C C G G G T G G C C    A G C A G C A G C C
A A C G C T C C A G    T G G C T C C A A G
G T C G G C A G A C    A G C C A G G G A A
G A C G C G C A A C    A G G T C A G G G A 400
T G G C A T G C A A    G A C C A C C A C C
A C C A T C A G C T    C T A A G C G A A T

C G T C C G T C G T    C C A T C C C T A C
C G A G T T T G A A    G A A A C C T A T T
A T C C T A A G A A    G G T C T G G G T G 500
C C A A G T C C C C    A C C G T C C T C C
G C C G A G G C T A    T C T C C A A C T G
T T C A C C G A A G    A G T G T C T C A A
G T T C T G C G C C    T C C A A G C A G G
A G G C C G T G G A    G A A G G C G C T G 600
A A C G A G G A G A    A G G T G G C C T A
C G A C T G C A G C    C C C A A C A A G A
A C A G G T A C C T    G A A C G T G G T C
C T G A A C A C C C    T C A A G A G A C T
G A A G G G C C T G    A C C C C C A G C T 700
C C A T G C C G G G    C C T C A G C A G G
G C C G C C C T G T    A C A G C C G C C T
C C A G G A G T T C    C T G C T C A G C C
A G G A C C A G C T    C A A G G A G A A C
G G C T A C C C C T    T C C C G C A C C C 800
C G A G C G G C C C    G G A G G C G C C G
T C C T C T T C A C    T G G C C A G G G G
A A G G G G C C C G    G C G A C T C C T C
C T G C A G G G T C    T G C T G C C G T T
G T G G C A C C G A    G T A C C T G G T G 900
T C C T C C T C G G    G C C G C T G T G T
A C G C G A C C A G    T T G T G T T A T T
A T C A C T G G G G    G C G G G T C C G C

T C G A G C C A G G    T G G C T G G A G G
C C G G G T T A G C    C A G T A C A C C T 1000
G C T G T G C A G C    T G C T C C T G G C
T C T G T G G G C T    G C C A G G T G G C
A A A G C A G C A C    G T G C G G G A C G
G C C G C A A G G A    C A G C C T C G A T
G G C T T C G T G G    A G A C C T T C A A 1100
G A A A G A G T T G    T C C A G A G A C G
C T T A T C C A G G    A A T C T A C G C C
T T G G A C T G T G    A G A T G T G C T A
C A C C A C G C A T    G G C C T A G A G C
T G A C C C G C G T    C A C C G T G G T G 1200
G A C G C C G A C A    T G C G A G T G G T
G T A C G A C A C C    T T C G T C A A G C
C C G A C A A C G A    G A T C G T G G A C
T A C A A C A C C A    G G T T T T C C G G
A G T C A C C G A G    G C C G A C G T C G 1300
C C A A G A C G A G    C A T C A C C T T G
C C C C A A G T G C    A A G C C A T C C T
G C T G A G C T T T    T T C A G C G C C C
A A A C C A T C C T    C A T C G G G C A C
A G C C T G G A G A    G C G A T C T G C T 1400
G G C C C T G A A G    C T C A T C C A C A
G C A C C G T G C T    G G A C A C G G C C
G T G C T C T T C C    C G C A C T A C C T
G G G T T T C C C C    T A C A A G C G T T

C C C T C A G G A A     T C T C G C G G C C 1500
G A C T A C C T G G     G A C A G A T C A T
C C A G G A C A G C     C A G G A C G G C C
A C A A G T G G A G     C G A G G A C G C A
A A C G C C T G C C     T G C A G C T G G T
G A T G T G G A A G     G T C C G A C A G C 1600
G C G C C C A G A T     C C A G C C A C G C
C A C C G G T C C G     C C T C T C C C G C
C G C C C T G G C C     T G T C C T T G G C
C C C A G G C C C C     T T C C A C A A C C
G C C A T C A G T C     C C G A G A G C T C 1700
A C C C T G T C C A     C C T C G C C G C A
A G G C C A A A G A     A A C C G G A G C A
G T C G A C G G C A     G G A G A G G G C A
A A A A G C C A A G     A G T A A C C C C A
A C C G G C C A C T     C C C A G T C C C C 1800
C G G A A T C C C T     G C C G C G G A C C
C T C G G G C C T G     T C C C C A T C C C
T C T G C C C T T C     C C A G A C C T C T
G T C C T T C C A C     T A A T C G C C T C
C C G C A G C A C C     G A G C C A C C A C 1900
T C C C A G T C C C     C C G A G T C C C T
G C C G C G C C C C     C T C G C G C C T G
T C C A C A T C C C     T C T G C C C A T C
C G A G A C C T C T     G T C C T T A C A C
C A C T A G C C A C     C C C A C G T G G G 2000

ACTTCCATGG CCTCTGAGTA
CAAGGCCAGC CCCCCGGCCC
ACCAGCTTTC TGAATGTGTG
CTTACCTGTT 2070.

2. Protéine GOR gab recombinante consistant en la séquence d'acides aminés suivante :

EFPLPSHQPA | QSFGLRVPQM
HNQASAFVDI | QAEPQNRGPA
VPPACLKMVT | EASYFPRQRG
SACCLPAAPR | LTERPSGVRI
SAPRKRKTIA | QSSSPCLVTG | 100
CTDAKRTRVA | SSS QRSSGSK
VGTQPGKTRN | RSGMACKTTT
TISSKRIVRR | PSLPSLKKPI
ILRRSGCQVP | TVLRRGYLQL
FTEECLKFCA | SKQEAVEKAL | 200
NEEKVAYDCS | PNKNRYLNVV
LNTLKRLKGL | TPSSMPGLSR
AALYSRLQEF | LLSQDQLKEN
GUPFPHPERP | GGAVLFTGQG
KGPGDSSCRV | CCRCGTEYLV | 300
SSSGRCVRDQ | LCYYHWGRVR
SSQVAGGRVS | QYTCCAAAPG
SVDCQVAKQH | VRDGRKDSLD
GRVETFKKEL | SRDAYPGIYA

L D C E M C Y [?] T H G L E L T R V T V V 400

D A D M R V V Y D T F V K P D N E I V D

Y N T R F S G V T E A D V A K T S I T L

P Q V Q A I L L S F F S A Q T I L I G H

S L E S D L L A L K L I H S T V L D T A

V L E P H Y L G F P Y K R S L R N L A A 500

D Y L G Q I I Q D S Q D G H N S S E D A

N A C L Q L V M W K V R Q R A Q I Q P R

H R S A S P A A L A C P W P Q A P S T T

A I S P E S S P C P P R R K A K E T G A
564

V D G R R G Q K A K S N P N R P L P V P 600

R N P C R G P S G L S P S L C P S Q T S

V L P L I A S R S T E P P L P V P R V P

A A P P R A C P H P S A H P R P L S L H

H 661.

3. Peptide d'hépatite non-A, non-B comprenant une chaîne d'acides aminés de dix acides aminés consécutifs ou plus choisis parmi les acides aminés numérotés de 1 à 564 de la protéine selon la revendication 2.

4. Anticorps monoclonal ou polyclonal spécifique de l'hépatite non-A, non-B réactif avec un antigène choisi parmi les antigènes comportant les acides aminés numérotés de 1 à 564 de la séquence de protéine selon la revendication 2.

5. Anticorps monoclonal ou polyclonal spécifique de l'hépatite non-A, non-B réactif avec un antigène comportant un peptide selon la revendication 3.

6. Kit de test de diagnostic d'hépatite non-A, non-B pour analyser des échantillons en vue de détecter la présence d'anticorps dirigés contre un antigène de l'hépatite non-A, non-B, comprenant la protéine antigène de l'hépatite non-A, non-B selon la revendication 2 liée à un substrat solide.

7. Kit de test de diagnostic d'hépatite non-A, non-B selon la revendication 6, comprenant en outre des anticorps marqués anti IgG ou IgM humaine.

8. Kit de test de diagnostic d'hépatite non-A, non-B pour analyser des échantillons en vue de détecter la présence d'anticorps dirigés contre un antigène de l'hépatite non-A, non-B, comprenant des anticorps marqués anti IgG ou IgM humaine et le peptide de l'antigène de l'hépatite non-A, non-B selon la revendication 3 lié à un substrat solide.

9. Kit de test de diagnostic d'hépatite non-A, non-B selon la revendication 8, comprenant en outre des anticorps marqués anti IgG ou IgM humaine.

10. Kit de test de diagnostic d'hépatite non-A, non-B pour analyser des échantillons en vue de détecter la présence d'un antigène de l'hépatite non-A, non-B, comprenant l'anticorps monoclonal ou polyclonal spécifique de l'hépatite non-A, non-B selon la revendication 4 lié à un substrat solide, lesdits anticorps étant marqués.

11. Kit de test de diagnostic d'hépatite non-A, non-B pour analyser des échantillons en vue de détecter la présence d'un antigène de l'hépatite non-A, non-B, comprenant l'anticorps monoclonal ou polyclonal spécifique de l'hépatite non-A, non-B selon la revendication 5 lié à un substrat solide, lesdits anticorps étant marqués.

12. Procédé de détection des anticorps de l'hépatite non-A, non-B dans un échantillon comprenant les étapes suivantes :
(a) réaction de l'échantillon avec la protéine d'hépatite non-A, non-B selon la revendication 2 liée à un substrat solide dans des conditions permettant la formation d'un complexe antigène-anticorps;
(b) réaction du produit de l'étape (a) avec des anticorps marqués anti IgG ou IgM humaine; et
(c) détection de la présence d'anticorps marqués anti IgG ou IgM humaine liés au complexe antigène-anticorps.

13. Procédé de détection des anticorps de l'hépatite non-A, non-B dans un échantillon comprenant les étapes suivantes :
(a) réaction de l'échantillon avec le peptide d'hépatite non-A, non-B selon la revendication 3 lié à un substrat solide dans des conditions permettant la formation d'un complexe antigène-anticorps;
(b) réaction du produit de l'étape (a) avec des anticorps marqués anti IgG ou IgM humaine; et
(c) détection de la présence d'anticorps marqués anti IgG ou IgM humaine liés au complexe antigène-anticorps.

14. Procédé de détection des antigènes de l'hépatite non-A, non-B dans un échantillon comprenant les étapes suivantes :
(a) réaction de l'échantillon avec l'anticorps monoclonal ou polyclonal spécifique de l'hépatite non-A, non-B selon la revendication 4 lié à un substrat solide dans des conditions permettant la formation d'un complexe antigène-anticorps;
(b) réaction du produit de l'étape (a) avec l'anticorps monoclonal ou polyclonal spécifique de l'hépatite non-A, non-B selon la revendication 4 qui est marqué; et
(c) détection de la présence d'anticorps marqués.

15. Procédé de détection des antigènes de l'hépatite non-A, non-B dans un échantillon comprenant les étapes suivantes :
(a) réaction de l'échantillon avec l'anticorps monoclonal ou polyclonal spécifique de l'hépatite non-A, non-B selon la revendication 5 lié à un substrat solide dans des conditions permettant la formation d'un complexe antigène-anticorps;
(b) réaction du produit de l'étape (a) avec l'anticorps monoclonal ou polyclonal spécifique de l'hépatite non-A, non-B selon la revendication 5 qui est marqué; et
(c) détection de la présence d'anticorps marqués.

16. Utilisation d'une protéine selon la revendication 2 pour la préparation d'un anticorps polyclonal spécifique de l'hépatite non-A, non-B réactif avec une protéine selon la revendication 2.

17. Utilisation d'une protéine selon la revendication 2, dans la production d'un anticorps polyclonal spécifique de l'hépatite non-A, non-B réactif avec cette protéine.

18. Utilisation d'une protéine selon la revendication 3 pour la préparation d'un anticorps polyclonal spécifique de l'hépatite non-A, non-B réactif avec une protéine selon la revendication 3.

19. Utilisation d'une protéine selon la revendication 3, dans la production d'un anticorps polyclonal spécifique de l'hépatite non-A, non-B réactif avec cette protéine.
